(19) Europäisches Patentamt
European Patent Office
Office européen des brevets

(11) Publication number: **0 060 904**
**B1**

(12) # EUROPEAN PATENT SPECIFICATION

(45) Date of publication of patent specification: **10.09.86**

(21) Application number: **81102099.9**

(22) Date of filing: **20.03.81**

(51) Int. Cl.[4]: **C 07 D 301/02,** C 07 D 303/04, C 07 D 303/08, C 07 D 303/22, C 07 C 17/00, C 07 C 19/02

(54) **A process for making vicinal epoxides and alkyl halides or alkylene dihalides from carbonate esters.**

(43) Date of publication of application:
**29.09.82 Bulletin 82/39**

(45) Publication of the grant of the patent:
**10.09.86 Bulletin 86/37**

(84) Designated Contracting States:
**BE DE FR GB NL**

(56) References cited:
**US-A-3 261 848**
**US-A-4 069 234**

**Houben-Weyl Band VIII, page 107,**
**Comprehensive Organic Chemistry Vol. 2, page 1075**

(73) Proprietor: **THE DOW CHEMICAL COMPANY**
**2030 Dow Center Abbott Road P.O. Box 1967**
**Midland, MI 48640 (US)**

(72) Inventor: **Renga, James Michael**
**1315 Wildwood**
**Midland Michigan (US)**
Inventor: **Emmons, Albert Henry**
**521 Costly**
**Midland Michigan (US)**

(74) Representative: **Casalonga, Axel et al**
**BUREAU D.A. CASALONGA OFFICE JOSSE & PETIT Morassistrasse 8**
**D-8000 München 5 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a new process for making vicinal epoxides and alkyl halides or alkylene dihalides.

Vicinal epoxides are valuable chemical intermediates and monomers useful in making epoxy adhesives and various heat- and solvent-resistant polymers. A well-known process for making vicinal epoxides from olefins involves the oxidation of the olefinic double bond with aqueous chlorine to form the chlorohydrin and reaction of the chlorohydrin with a base to make the epoxide. However, a major disadvantage of this process is the production of an equivalent of HCl from the aqueous oxychlorination step and another equivalent of inorganic chloride from the reaction of the base with the chlorohydrin intermediate. In the case of epichlorohydrin, the conventional preparation uses the same chemistry with the added initial step of chlorinating propylene to allyl chloride which produces an additional equivalent of HCl.

Ethylene oxide is prepared by oxidizing ethylene with molecular oxygen over a silver catalyst. However, this method is not applicable to other olefins because of low selectivity and the formation of by-products. Another method using oxygen involves oxidizing a hydrocarbon such as isobutane or isopropylbenzene with air to the corresponding tertiary hydroperoxide and then reacting the hydroperoxide with an olefin in the presence of a transition metal catalyst. A disadvantage of this process is the formation of co-product alcohol which must be sold or recycled.

Hydrogen peroxide and peroxy acids are other reagents which have been used to epoxidize olefins. Chemical and economic disadvantages of such methods have precluded their use on a large scale.

U.S. patent 3,261,848 teaches preparation of the unsaturated epoxide 1,2 - divinyl ethylene oxide by caustic treatment of the chloroester 3 - chloro - 4 - acetoxy 1,5 - hexadiene.

It is known that cyclic carbonates can be decomposed to form epoxides in the presence of various catalysts. Such a process particularly directed to the preparation of propylene oxide by decomposition of propylene carbonate in the presence of a sulfonium or phosphonium halide or any of certain metal salts is described in U.S. Patent 4,069,234, issued to Wu January 17, 1978.

The present invention is a process for making a vicinal epoxide and an alkyl halide or alkylene dihalide comprising contacting a carbonate ester of the formula

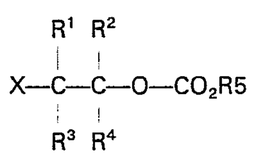

(I)

or

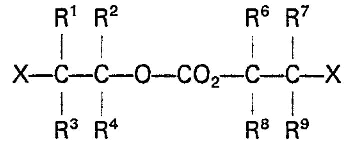

(II)

wherein $R^1$ to $R^4$ and $R^6$ to $R^9$ are each hydrogen, an alkyl group of 1—20 carbon atoms, a cycloalkyl or alkylcycloalkyl group of 5—10 carbon atoms, an aromatic hydrocarbon group of 6—10 carbon atoms or $—CH_2X$, $R^5$ is alkyl, $R^6$ to $R^9$ can each be $—CH_2Y$, and each of the pairs $R^1$, $R^2$ and $R^6$, $R^7$ may together form an alkylene group of 3—6 carbon atoms, each X is Cl or Br, and Y is an alkoxy or aroxy group, with a quaternary ammonium or phosphonium salt catalyst at 150°C—250°C and separating said epoxide from the reaction mixture.

In the first aspect of the present invention, the simple alkylene and cycloalkylene oxides and their aromatic and halogen-substituted derivatives, can be made in good yield by heating an unsymmetrical β-haloalkyl carbonate of formula (I) as described above. The products of this decomposition are $CO_2$, the halide $R^5X$, and the epoxide of the formula

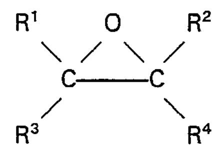

wherein X, each of $R^1$, $R^2$, $R^3$, $R^4$, and $—CH_2X$, are as described above, and $R^5$ is an alkyl group, preferably a lower alkyl group $R^3$ and $R^4$ being preferably hydrogen. This process produces two useful products, the alkyl halide $R^5X$ and the epoxide, assuming $CO_2$ to be a waste product. The structure of the starting β-haloalkyl carbonate, therefore, is normally designed to produce not only the desired epoxide, but also a particular useful alkyl halide which has a boiling point sufficiently different from the epoxide to facilitate easy and complete separation of these products.

Regarding the second aspect of the present invention, when $R^5$ in the carbonate formula is also a β-haloalkyl group as defined therein, the products of the decomposition reaction then are a vicinal epoxide, $CO_2$, and an alkylene dihalide. The alkylene dihalide produced has a boiling point sufficiently different from the epoxide so that their separation is easily achieved. When the two haloalkyl groups in the carbonate molecule are of unequal size, for example, as in a haloethyl halopropyl carbonate where the epoxide product might reasonably be expected to be a mixture of about equal amounts of the two possible compounds, surprisingly, the epoxide with the longer carbon chain, propylene oxide in the example cited, is produced as by far the predominant epoxide product, a molar excess of up to 10—15/1 being typical.

The carbonate starting material for the second

aspect of the invention is that of formula (II) where each R group and X are described above, and Y is an alkoxy group, preferably of 1—4 carbon atoms, or an aroxy group such as a phenol or bisphenol residue. Thus the primary epoxide product can be

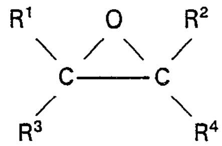

or

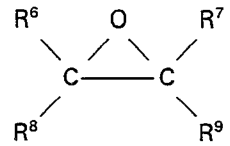

while the principal alkylene dihalide co-product would be either

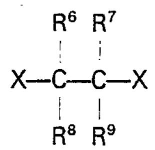

or

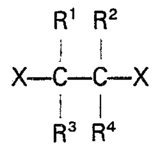

respectively, depending upon the relative sizes of the haloalkyl groups in the starting carbonate.

Process parameters

The following parameters are common to both aspects of the present invention:

1. The decomposition reaction takes place in the presence of the quaternary salt catalyst at some rate at any temperature from about 150°C to about 250°C. Reaction times can range from 0.001 hour to about 10 hours depending on the structure of the carbonate, the temperature, and the nature and amount of the catalyst.

2. Substantially any quaternary ammonium or phosphonium salt can catalyze the decomposition reaction. Preferably, these salts have the general formula $R_4AZ$ where each R is a hydrocarbon moiety; A is a quaternized nitrogen or phosphorus atom; and Z is an inert (i.e., inert in this process) neutralizing anion which may be inorganic, e.g., chlorine, bromide iodide, bicarbonate or sulfate: or Z may be an organic ion such as formate, acetate, benzoate, phenate, or bisphenolate. The R groups may be alkyl, aryl, alkaryl, aralkyl, or cycloalkyl. Also, two R groups may combine to form a heterocyclic ring. Illustrative quaternary salt catalysts are tetrabutylammonium bromide, benzyltriethylammonium chloride, N-methylpyridinium chloride, N,N - dibutylmorpholinium iodide, N - propylpyrrolium chloride, tetrabutylphosphonium bromide, tributylmethylphosphonium formate, tetrapropylphosphonium bisulfate, and corresponding ammonium and phosphonium salts with these and other such inorganic and organic neutralizing anions as described above. The catalytic salt may be added as such to the reaction mixture or it may be formed *in situ*.

(In the second aspect of the present invention, amine and phosphine salts such as tributylamine hydrochloride which are a form of quaternary salt will catalyze the reaction although these are generally less desirable in the reaction mixture. Similarly, when a nitrogen-containing solvent such as N,N - dimethylformamide or N,N - dimethylacetamide is employed in the process, the small amount of quaternary salt formed by interaction of the amide nitrogen atom with the halide reactant, or dihalide product, is sufficient to catalyze the decomposition reaction.)

3. Although any significant amount of such a quaternary salt will catalyze the decomposition reaction to some extent, for practical reasons in batch operations, it is preferred to use about 0.1—10 mole percent of the salt based on the carbonate. More quaternary salt catalyst can be used but the excess confers little added advantage and may in fact be disadvantageous.

4. In a mode of the invention particularly adapted to continuous operation, one or more R groups may be pendant methylene groups from a resin matrix so that the quaternary salt is a salt form of a strong base anion-exchange resin for example a strong base anion-exchange resin having quaternary ammonium halide functionalities or the phosphonium equivalents of such quaternary ammonium-substituted resins. In such a continuous operation of the process, the β-haloalkyl carbonate starting material is passed at an appropriate flow rate through a bed of the strong base anion resin maintained at a suitable temperature within the limits previously defined. Similarly, a solid particulate catalyst can be formed by depositing a quaternary ammonium or phosphonium salt as described above on silica, alumina, clay, a zeolite, or other such inert support.

5. A reaction solvent or diluent is usually of no advantage and the process is ordinarily run in the absence of such an inert additive. In some cases, however, a solvent may be of some advantage. Inert solvents suitable for use include hydrocarbons such as toluene, xylene, and decane; glycol diethers such as dimethyloxyethane, substituted amides such as N,N - dimethylformamide, and cyclic compounds such as tetrahydrofuran and sulfolane.

6. In the preparation of higher boiling epoxides particularly, separation of the epoxide product may be facilitated by running the reaction under appropriately reduced pressure or by passing a stream of nitrogen or other inert gas through or over the reaction mixture.

The β-haloalkyl alkyl carbonate starting materials for the first aspect of the present invention can be prepared by any of several generally known procedures. Pechukas, USP 2,518,058 describes the reaction of an epoxide with a haloformate to make a corresponding β-haloalkyl alkyl carbonate. These mixed carbonate esters can also be made by the acid-catalyzed transesterification reaction of a halohydrin with a dialkyl carbonate. For example 2-chloroethyl methyl carbonate is produced by the reaction of dimethyl carbonate with ethylene chlorohydrin and 1 - chloro - 2 - propyl ethyl carbonate can be made by reacting diethyl carbonate with 1 - chloro - 2 - propyl alcohol.

Variations of this method can be used to make particular halogenated alkyl carbonate esters. Corresponding monohalo- and dihalopropyl carbonates, for example, can be made by first reacting allyl alcohol with a dialkyl carbonate and then adding hydrogen halide or halogen to the olefinic double bond in the allyl alkyl carbonate product.

The β-halogenated alkyl carbonate starting materials for the second aspect of the present invention can be prepared by several known procedures. The reaction of a chloroformate with an alcohol conventionally used for the preparation of carbonate esters is readily adapted to the preparation of these halogenated carbonates by using the appropriate halogenated alcohol and halogenated alkyl chloroformate reactants. Symmetrical bis(haloalkyl) carbonates in particular can be made by the strong acid catalyzed transesterification reaction of a halogenated alcohol in excess with a dialkyl carbonate. Some of these carbonates can also be made by using an appropriate unsaturated alcohol in the transesterification reaction and then adding halogen or hydrogen halide to the unsaturated ester product. A method recently described in Japanese Patent 46,921/78 whereby a cyclic carbonate such as ethylene carbonate or propylene carbonate is reacted at moderate temperature with an olefin and chlorine or bromine in equal molar amounts is another means of obtaining the halogenated alkyl carbonate starting materials of this invention. By using ethylene carbonate (which is made from ethylene oxide) and an olefin other than ethylene as reactants in the cited Japanese process to make the haloalkyl carbonate starting material of this invention, the present process becomes essentially a means for transferring the epoxide value of ethylene oxide to higher olefins using organic carbonates as intermediates.

As noted previously, when the two haloalkyl groups in the carbonate molecule are different, two different epoxides and two different alkylene dihalides are produced by the decomposition, the proportions of these products depending upon the relative sizes of the two alkyl groups. Thus, when 2-chloroethyl 1 - chloro - 1 - propyl carbonate is subjected to the conditions of the decomposition process, the principal epoxide and dihalide products are propylene oxide and ethylene dichloride with minor amounts of ethylene oxide and propylene dichloride being formed. Ordinarily, conventional distillation of the mixed products provides effective separation of the individual components as pure compounds.

The advantage of the second aspect of the present invention over the first is essentially the greater economic value of the alkylene dihalide product as compared to the value of the lower alkyl halide product. This process is ordinarily of most interest as a means of producing epoxides of higher molecular weight than ethylene oxide such a propylene oxide, epichlorohydrin, and alkyl or aryl glycidyl ethers. It has particular advantage as mentioned elsewhere in that the halide coproduct is not a useless inorganic waster material but rather an economically desirable alkylene dihalide. The minor by-products, usually ethylene oxide and another alkylene dihalide, are readily recoverable and, of course, are also valuable compounds.

The following Examples 1—17 illustrate both aspects of the present invention.

Example 1

A mixture of 4.57 g of 1 - chloro - 2 - propyl methyl carbonate (contained 20—30 percent of the 2 - chloro - 1 - propyl isomeric ester) and 0.034 g of tetrabutylphosphonium bromide in a 10 ml reaction flask was heated by an oil bath at 180°C—185°C for 2 hours. The flask was equipped with a magnetic stirrer, a condenser, and a receiver plus a trap, each of the latter containing 10 g of chloroform cooled to −60°C. After 2 hours of heating, the residue in the reaction flask amounted to 0.23 g of material which contained less than 5 percent starting carbonate. The receiver and trap had gained a total of 2.5 g of reaction products which were determined by nuclear magnetic resonance spectroscopic and chromatographic analysis to be a mixture of propylene oxide and methyl chloride, some methyl chloride having been lost because of its high volatility. The conversion of chloropropyl methyl carbonate was nearly 100 percent and the analyses indicated a yield of about 95 percent of the theoretical for propylene oxide.

Examples 2—3

The procedure of Example 1 was repeated twice using 0.027 g of tetrabutylammonium chloride and 0.037 g of tetrabutylammonium iodide, respectively, in place of the phosphonium salt catalyst. In each case, the yield of propylene oxide was 97—99 percent of the theoretical amount but the conversion of starting carbonate was relatively low, about 20 percent and 25 percent respectively.

Example 4

The procedure of the above examples was repeated using 0.5 g of anion-exchange resin as the catalyst. The resin contained 40—50 percent water. The resin is a strong base anion resin consisting of macroporous cross-linked styrene polymer matrix having pendant quaternary ammonium chloride functionalities. After 2.5 hours of heating time, about 99 percent of the carbonate had been decomposed to form 95 percent of the theoretical quantity of propylene oxide.

Example 5

A mixture of 4.16 g of 2-chloroethyl methyl carbonate and 0.034 g of tetrabutylphosphonium bromide was heated at 180°C for 3 hours in the apparatus previously described. A carbonate conversion of 99.7 percent was obtained with an 89 percent yield of ethylene oxide.

Example 6

In the same way as in Example 5, a mixture of 5.49 g of 2-bromoethyl methyl carbonate and 0.034 g of tetrabutylphosphonium bromide was heated for 6 hours at 200°C to produce a carbonate conversion of 100 percent and an 88 percent selectivity to ethylene oxide and methyl bromide.

Example 7

A mixture of 3.34 g of 1 - chloro - 2 - octyl methyl carbonate (containing 21 percent of the corresponding 2 - chloro - 1 - octyl ester) and 0.024 g of tetrabutylphosphonium formate was heated as above at 200°C—205°C for 2 hours at reduced pressure $2.7\times10^4$ Pa (200 mm Hg). An isolated yield of 96 percent of theory of 1,2-epoxyoctane was collected in the receiver.

Example 8

A mixture of 2.89 g of 2 - chorocyclohexyl methyl carbonate and 0.039 g of tetrabutylphosphonium salt of Bisphenol A was heated at 200°C—205°C for 1.5 hours. A yield of 1.34 g of 1,2 - epoxycyclohexane was collected in the receiver.

Example 9

In a procedure similar to that used in Example 7, a mixture of 3.89 g of 2 - bromo - 1 - phenylethyl methyl carbonate and 0.024 g of tetrabutylphosphonium formate was heated at 180°C for 2 hours at $6.7\times10^3$ Pa (50 mm Hg) absolute pressure. The product condensed in the receiver was 1.58 g of a mixture containing 40 percent styrene oxide and 60 percent phenylacetaldehyde.

Example 10

The reduced pressure technique of Examples 7 and 9 was followed in heating a mixture of 5.61 g of 1,3 - dichloro - 2 - propyl methyl carbonate and 0.078 g of the tetrabutylphosphonium Bisphenol A salt used in Example 8. After 2 hours at 195°C—200°C and $1.3\times10^4$ Pa (100 mm Hg) absolute pressure, 2.85 g of 88 percent pure epichlorohydrin had condensed in the receiver.

Example 11

To a 4-neck 50 ml reaction flask equipped with a mechanical stirrer, addition funnel, distillation head, and nitrogen inlet there was added 0.24 g of tetrabutylphosphonium formate and the flask was heated to 185°C—190°C with a stream of 30 ml/min. of nitrogen passing through while 2.81 g of 2,3 - dichloro - 1 - propyl methyl carbonate was added over a period of 30 minutes. Analyses of 1.4 g of condensed effluent in the receiver cooled by solid $CO_2$ and 0.47 g of residue indicated a 90—95 percent conversion of carbonate with a 50—60 percent yield of epichlorohydrin.

Example 12

A mixture of 3.02 g (0.015 g mole) of 2 - chloro - ethyl 1 - chloro - 2 - propyl carbonate and 0.051 g (0.00015 g mole) of tetrabutylphosphonium bromide was heated at 190°C—192°C in a 10 ml reaction flask equipped with magnetic stirrer and a distillation head connected to a receiver containing 11.9 g of chloroform cooled to −60°C. The 2 - chloroethyl 1 - chloro - 2 - propyl carbonate starting material was prepared by reacting 1.5 moles of ethylene carbonate with about two moles each of propylene and chlorine at about room temperature as shown in Japanese Patent 46,921/78. After 3 hours of heating, a residue of 0.20 g remained in the reaction flask and the receiver had gained 2.15 g in total weight. Nuclear magnetic resonance spectroscopic and chromatographic analyses of the reaction products indicated that 97 percent of the starting carbonate had been converted to a mixture of 83 percent of the theoretical quantity of propylene oxide and 6 percent of the theoretical amount of ethylene oxide together with corresponding yields of 1,2-dichloroethane and 1,2-dichloropropane, respectively.

Example 13

The procedure of Example 12 was repeated except for using five times the amount (0.255 g) of tetrabutylphosphonium bromide catalyst and heating the reaction mixture only one hour at the indicated temperature. A 97 percent conversion of the starting carbonate was obtained with 74 percent yield of propylene oxide and 6 percent yield of ethylene oxide together with 86 percent yield of ethylene dichloride and 9 percent yield of propylene dichloride.

Example 14

The procedure of Example 12 was repeated except that 0.25 g of the same ion-exchange resin was used as the catalyst.

After 2 hours of heating, 33 percent of the starting carbonate had been converted to a mixture of propylene oxide in 56 percent yield and ethylene oxide in 5 percent yield plus a 76 percent yield of ethylene dichloride and a 12 percent yield of propylene dichloride.

Example 15

To a 25-ml reaction flask equipped with an addition funnel and a distillation head with receiver there was added 0.54 g of the mono(tetrabutylphosphonium) salt of Bisphenol A complexed with a molecule of the free bisphenol. The flask was heated to 190°C—192°C as 3.53 g (0.015 g mole) of a 2-chloroethyl 1,3 - dichloro - 2 - propyl carbonate was added portionwise over 2 hours with the flask and receiver maintained at $2\times10^4$ Pa (150 mm Hg) absolute pressure. The carbonate starting material was made by reacting 1,3 - dichloro - 2 - propanol with 2 - chloroethyl chloroformate under conventional alcohol chloroformate reaction conditions. The receiver was cooled with solid $CO_2$. Analyses of the 2.61 g condensed effluent in the receiver and the 0.71 g of residue in

the reaction flask indicated about 95 percent conversion of the starting carbonate with about 50—55 percent yield of epichlorohydrin.

Example 16

Following the procedure of Example 15, 3 - tert - butoxy - 1 - chloro - 2 - propyl 2 - chloroethyl carbonate (from the reaction of 3 - tert - butoxy - 1 - chloro - 2 - propanol with 2 - chloroethyl chloroformate) is heated in the presence of tetrabutylphosphonium bromide to produce tert-butyl glycidyl ether and ethylene dichloride as the principal reaction products.

Example 17

By reacting 3 - phenoxy - 1 - chloro - 2 - propanol with 2 - chloroethyl chloroformate according to conventional chloroformate ester reaction procedures, 2 - chloroethyl 3 - phenoxy - 1 - chloro - 2 - propyl carbonate is obtained. When the latter compound is heated in the presence of a phosphonium or ammonium salt catalyst as shown in the foregoing examples, phenyl, glycidyl ether is produced as the principal epoxide product.

When the chlorinated carbonate ester starting materials shown in Examples 12—17 are replaced by the corresponding bromo or mixed bromo chloro esters, similar results are obtained by following the indicated procedures. For example, 2 - bromoethyl 1 - bromo - 2 - propyl carbonate is thereby decomposed to form propylene oxide and ethylene dibromide as the principal epoxide and dihalide products, while 2 - bromoethyl 1 - chloro - 2 - propyl carbonate decomposes in the same way to produce propylene oxide and 1 - bromo - 2 - chloroethane as the principal reaction products.

**Claims**

1. A process for making a vicinal epoxide and simultaneously an alkyl halide or alkylene dihalide comprising contacting a carbonate ester of the formula

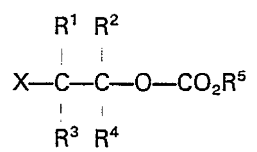

(I)

or

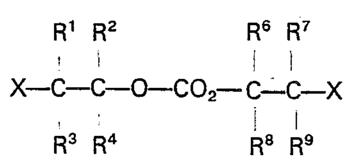

(II)

wherein $R^1$ to $R^4$ and $R^6$ to $R^9$ are each hydrogen an alkyl group of 1—20 carbon atoms, a cycloalkyl or alkyl-cycloalkyl group of 5—10 carbon atoms, an aromatic hydrocarbon group of 6—10 carbon atoms or $—CH_2X$, $R^5$ is alkyl, $R^6$ to $R^9$ can each be $—CH_2Y$, and each of the pairs $R^1$, $R^2$ and $R^6$, $R^7$ may together form an alkylene group of 3—6 carbon atoms, each X is Cl or Br, and Y is an alkoxy or aroxy group, with a quaternary ammonium or phosphonium salt catalyst at 150°C—250°C and separating from the reaction mixture an epoxide of the formula

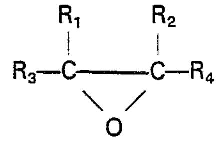

plus an alkyl halide $R_5X$ if a carbonate ester of formula (I) is used, or an epoxide of formula

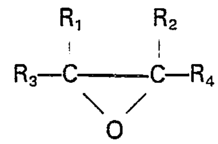

plus an alkylene dihalide of formula

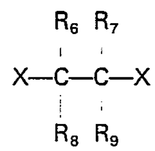

and/or an epoxide of formula

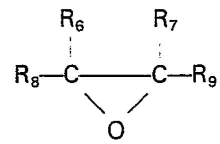

plus an alkylene dihalide of formula

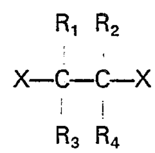

if a carbonate ester of formula (II) is used, each R group, X and Y having the above-mentioned meanings.

2. The process of claim 1 wherein the starting material is the compound of formula (I).

3. The process of claim 1 wherein the starting material is the compound of formula (II).

4. The process of claim 1 or 2 wherein $R^3$ and $R^4$ are hydrogen and $R^5$ is a lower alkyl group.

5. The process of claim 4 wherein $R^1$ is hydrogen, $R^2$ is a phenyl group, and the epoxide product is styrene oxide.

6. The process of claim 4 wherein $R^1$ and $R^2$ together form a tetramethylene group and the epoxide product is cyclohexene oxide.

7. The process of claim 4 wherein one of $R^1$ and $R^2$ is a hexyl group and the other is hydrogen and the epoxide product is 1,2-epoxyoctane.

8. The process of claim 4 wherein one of $R^1$ and $R^2$ is a methyl group and the other is hydrogen and the principal epoxide product is propylene oxide.

9. The process of claim 4 wherein one of $R^1$ and $R^2$ is a chloromethyl group and the other is hydrogen and the principal epoxide product is epichlorohydrin.

10. The process of claim 3 wherein $R^9$ is a lower alkoxymethyl group, $R^6$, $R^7$ and $R^8$ are hydrogen, and the principal epoxide product is a lower alkyl glycidyl ether.

11. The process of claim 10 wherein $R^9$ is a tert-butoxymethyl group and the principal epoxide product is tert-butyl glycidyl ether.

12. The process of claim 3 wherein $R^9$ is an aroxymethyl group, $R^6$, $R^7$ and $R^8$ are hydrogen, and the principal epoxide product is the aryl glycidyl ether.

13. The process of claim 12 wherein $R^9$ is a phenoxymethyl group and the principal epoxide product is phenyl glycidyl ether.

14. The process of anyone of claims 1 to 13 wherein the salt catalyst is a strong base anion-exchange resin having quaternary ammonium halide functionalities.

## Patentansprüche

1. Verfahren Herstellung eines vizinalen Epoxids und gleichzeitig eines Alkylhalogenids bzw. Alkylendihalogenids, das darin besteht, daß man einen Kohlensäureester der Formel

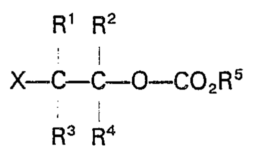
(I)

oder

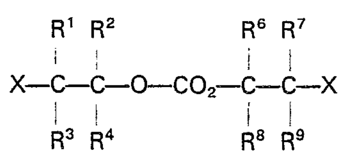
(II)

worin $R^1$ bis $R^4$ und $R^6$ bis $R^9$ jeweils Wasserstoff, eine Alkylgruppe mit 1 bis 20 Kohlenstoffatomen, eine Cyloalkyl- oder eine Alkylcycloalkylgruppe mit 5 bis 10 Kohlenstoffatomen, eine aromatische Kohlenwasserstoffgruppe mit 6 bis 10 Kohlenstoffatomen oder $—CH_2X$ sind, $R^5$ ein Alkyl ist, $R^6$ bis $R^9$ jeweils $—CH_2Y$ sind, und jedes der Paare $R^1$, $R^2$ bzw. $R^6$, $R^7$ zusammen eine Alkylengruppe mit 3 bis Kohlenstoffatomen bilden können, jedes X Cl oder Br ist, und Y eine Alkoxy- oder Aroxygruppe ist,

mit einem quaternären Ammonium- oder Phosphoniumsalzkatalysator bei 150—250°C in Kontakt bringt, und vom Reaktionsgemisch ein Epoxid der Formel

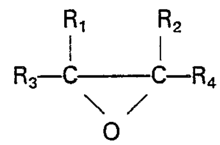

plus ein Alkylhalogenid $R_5X$ wenn ein Kohlensäureester der Formel (I) eingesetzt wird, bzw. ein Epoxid der Formel

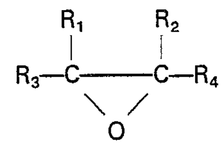

plus ein Alkylendihalogenid der Formel

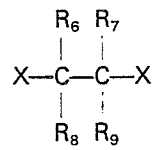

und/oder ein Epoxid der Formel

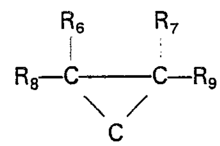

plus ein Alylendihalogenid der Formel

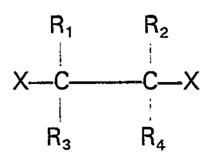

wenn ein Kohlensäureester der Formel (II) eingesetzt wird, wobei jede R-gruppe sowie X und Y die obigen Bedeutungen haben, abtrennt.

2. Verfahren gemäß Anspruch 1, wobei das Ausgangsmaterial die Verbindung der Formel (I) ist.

3. Verfahren gemäß Anspruch 1, wobei das Ausgangsmaterial die Verbindung der Formel (II) ist.

4. Verfahren gemäß Anspruch 1 oder 2, wobei es sich bei $R^3$ und $R^4$ um Wasserstoff, und bei $R^5$ um eine niedere Alkylgruppe handelt.

5. Verfahren gemäß Anspruch 4, wobei es sich bei $R^1$ um Wasserstoff, bei $R^2$ um eine Phenylgruppe, und beim Epoxidprodukt um Styroloxid handelt.

6. Verfahren gemäß Anspruch 4, wobei $R^1$ und $R^2$ zusammen eine Tetramethylengruppe bilden, und es sich beim Epoxidprodukt um Cyclohexenoxid handelt.

7. Verfahren gemäß Anspruch 4, wobei es sich bei $R^1$ bzw. $R^2$ um eine Hexylgruppe und bei dem jeweils anderen um Wasserstoff, und beim Epoxidprodukt um 1,2-Epoxyoctan handelt.

8. Verfahren gemäß Anspruch 4, wobei es sich

bei $R^1$ bzw. $R^2$ um eine Methylgruppe und bei dem jeweils anderen um Wasserstoff, und bei dem Hauptepoxidprodukt um Propylenoxid handelt.

9. Verfahren gemäß Anspruch 4, wobei es sich bei $R^1$ bzw. $R^2$ um eine Chlormethylgruppe und bei dem jeweils anderen um Wasserstoff, und bei dem Hauptepoxidprodukt um Epichlorhydrin handelt.

10. Verfahren gemäß Anspruch 3, wobei es sich bei $R^9$ um eine niedere Alkoxymethylgruppe, bei $R^6$, $R^7$ und $R^8$ um Wasserstoff, und beim Hauptepoxidprodukt um einen niederen Alkylglyzidyläther handelt.

11. Verfahren gemäß Anspruch 10, wobei es sich bei $R^9$ um eine tert. Butoxymethylgruppe, und beim Hauptepoxidprodukt um einen tert. Butylglyzidyläther handelt.

12. Verfahren gemäß Anspruch 3, wobei es sich bei $R^9$ um eine Aroxymethylgruppe, bei $R^6$, $R^7$ und $R^8$ um Wasserstoff, und beim Hauptepoxidprodukt um Arylglyzidyläther handelt.

13. Verfahren gemäß Anspruch 12, wobei es sich bei $R^9$ um eine Phenoxymethylgruppe, und beim Hauptepoxidprodukt um Phenylglyzidyläther handelt.

14. Verfahren gemäß einem beliebigen der Ansprüche 1 bis 13, wobei es sich bei dem Salzkatalysator um ein starkes basisches Anionaustauscherharz mit quaternären Ammoniumhalogenidfunktionen handelt.

## Revendications

1. Procédé pour produire un époxyde vicinal, et simultanément un halogénure d'alkyle ou dihalogénure d'alkylène qui comprend la mise en contact d'un ester carbonate de formule

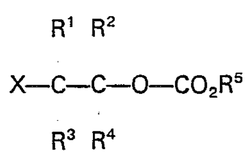 (I)

ou

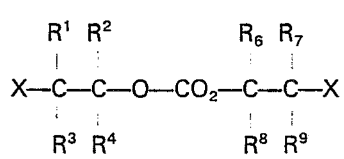 (II)

dans lesquelles chaque $R^1$ à $R^4$ et chaque $R^6$ à $R^9$ est un atome d'hydrogène ou un groupe alkyle de 1—20 atomes de carbone, un groupe cycloalkyle ou alkyl-cycloalkyle de 5—10 atomes de carbone, un groupe hydrocarboné aromatique de 6—10 atomes de carbone, ou un groupe $—CH_2X$, $R^5$ est un groupe alkyle, chaque $R^6$ à $R^9$ peut être un groupe $—CH_2Y$, et chacune des paires de groupes $R^1$, $R^2$ et $R^6$, $R^7$ peut former un groupe alkylène de 3—6 atomes de carbone, chaque X est un atome de chlore ou de brome, et Y est un group alcoxy ou aroxy, avec un sel de phosphonium ou d'ammonium quaternaire comme catalyseur, à 150°—250°C, et la séparation, à partir du mélange réactionnel, d'un époxyde de formule

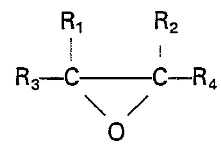

et d'un halogénure d'alkyle $R_5X$ si l'on utilise un ester carbonate de formule (I), ou d'un époxyde de formule

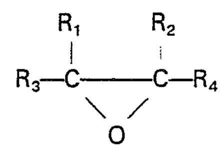

et d'un dihalogénure d'alkylène de formule

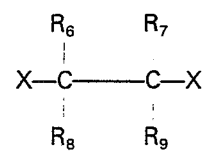

et/ou d'un époxyde de formule

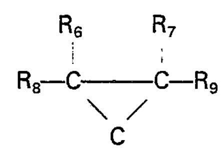

et d'un dihalogénure d'alkylène de formule

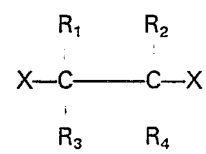

si on utilise un ester carbonate de formule (II), chaque groupe R, X et Y ayant les significations mentionnées ci-dessus.

2. Procédé conforme à la revendication 1, dans lequel le produit de départ est le composé de formule (I).

3. Procédé conforme à la revendication 1, dans lequel le produit de départ est le composé de formule (II).

4. Procédé conforme à l'une des revendications 1 ou 2, dans lequel $R^3$ et $R^4$ sont des atomes d'hydrogène et $R^5$ est un groupe alkyle inférieur.

5. Procédé conforme à la revendication 4, dans lequel $R^1$ est un atome d'hydrogène, $R^2$ est un groupe phényle, et le produit époxyde est l'oxyde de styrène.

6. Procédé conforme à la revendication 4, dans lequel $R^1$ et $R^2$ forment ensemble un groupe tétraméthylène, et le produit époxyde est l'oxyde de cyclohexène.

7. Procédé conforme à la revendication 4, dans lequel l'un de $R^1$ et $R^2$ est un groupe hexyle et

l'autre un atome d'hydrogène, et le produit époxyde est le 1,2-époxyoctane.

8. Procédé conforme à la revendication 4, dans lequel l'une de $R^1$ et $R^2$ est un groupe méthyle et l'autre un atome d'hydrogène, et le produit époxyde principal est l'oxyde de propylène.

9. Procédé conforme à la revendication 4, dans lequel l'un de $R^1$ et $R^2$ est un groupe choro-méthyle et l'autre un atome d'hydrogène, et le produit époxyde principal est l'épichlorhydrine.

10. Procédé conforme à la revendication 3, dans lequel $R^9$ est un groupe (alcoxy inférieur)-méthyle, $R^6$, $R^7$ et $R^8$ sont des atomes d'hydrogène, et le produit époxyde principal est un éther glycidique d'alkyle inférieur.

11. Procédé conforme à la revendication 10, dans lequel $R^9$ est un groupe tert-butoxyméthyle et le produit époxyde principal est l'éther glycidique de tert-butyle.

12. Procédé conforme à la revendication 3, dans lequel $R^9$ est un groupe aroxyméthyle, $R^6$, $R^7$ et $R^8$ sont des atomes d'hydrogène, et le produit époxyde principal est un éther glycidique d'aryle.

13. Procédé conforme à la revendication 12, dans lequel $R^9$ est un groupe phénoxyméthyle, et le produit époxyde principal est un éther glycidique de phényle.

14. Procédé conforme à l'une quelconque des revendications 1 à 13, dans lequel le sel catalyseur est unerésine échangeuse d'anions fortement basique, présentant des fonctions halogénure d'ammonium quaternaire.